# EUROPEAN PATENT APPLICATION

(11) **EP 2 180 003 A1**
(43) Date of publication of application: **28.04.2010**
(21) Application number: 08018374.2
(22) Date of filing: 21.10.2008
(51) Int. Cl.: C07F 9/38, A61P 35/00

(54) **Preparation of ibandronate trisodium**

(71) Applicant: Zentiva, k.s., 102 37 Praha 10 (CZ)
(72) Inventor: Jirman, Josef, 100 00 Praha 10 (CZ); Richter, Jindrich, 532 12 Pardubice (CZ); Havlicek, Jaroslav, 149 00 Praha 4 (CZ); Karliga, Bekir, Cerkezkoy, 59500 Tekirdag (TR)
(74) Representative: Jirotkova, Ivana

(57) **Abstract**

The trisodium salt of ibandronate of formula (I):

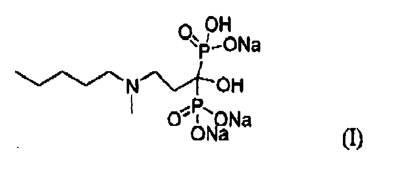

A process of preparing said salt comprises:
a) mixing 3-(methylpentylamino)propionic acid hydrochloride, phosphorous acid (in a 2.5-3.5 molar excess with respect to 3-(methylpentylamino)propionic acid hydrochloride), sunflower oil (in an amount of 6-9 volume parts for 1 weight part of 3-(methylpentylamino)propionic acid hydrochloride) and phosphorus trichloride (in a 3-4 molar excess with respect to 3-(methylpentylamino)propionic acid hydrochloride);
b) adding water (in an amount of 6-9 volume parts for 1 weight part of 3-(methylpentylamino)propionic acid hydrochloride) and after separation of the phases;
c) adjusting pH of the water phase to pH 12.5;
d) adding a water-miscible co-solvent and cooling down to precipitate the product or evaporate to dryness.

Another process for preparing the trisodium salt of ibandronate comprises admixing the monosodium salt of ibandronate or ibandronic acid or their solvates with water or a mixture water/sodium hydroxide and adjustment of pH to 12.5.

Various crystalline, semicrystalline and amorphous forms of trisodium ibandronate are disclosed.

## Description

### State of the art

The first bisphosphonates (originally called diphosphonates) were synthesized in 19th century (1). Geminal bisphosphonates are metabolically stable analogues of naturally occurring inorganic pyrophosphate. Like pyrophosphate, bisphosphonates have high affinity for bone mineral and high doses can modulate calcification both in vitro and in vivo. In contrast to pyrophosphate bisphosphonates are also orally active, although their low bioavailability often limits the usefulness of oral administration (2).
Ibandronic acid of formula 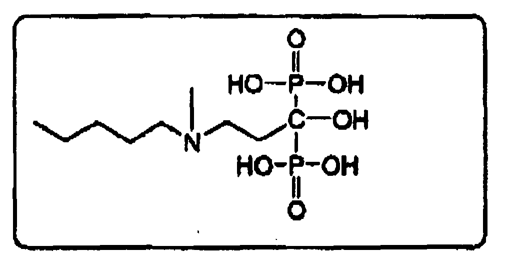 was developed by the Boehringer Mannheim company (3) as a powerful tool for treatment of osteoporosis.
Ibandronate sodium is a third-generation nitrogen-containing bisphosphonate characterized by an aliphatic tertiary amine side chain.
U.S. patent No. 4,972,814 discloses diphosphonic acid derivatives, process for preparation thereof and pharmaceutical compositions containing them.

Bioavailability of ibandronate in solid dosage form is also influenced by solid form or crystal form of the active pharmaceutical ingredient that has an impact to solubility. Ibandronic acid and ibandronate can form a lot of hydrates and solvates (3-9). Bioavailability and thus also solubility of bisphosphonate are solved by choosing a suitable very good soluble crystal form or amorphous form. Usually, more soluble in water are hydrates of ionic solid substances or amorphous materials, which are usually less stable. Ibandronic acid in solid form or ibandronate sodium can form solvates with a lot of pharmaceutically suitable and non suitable solvents (9). Solvents can compete with each other and can compete with water; therefore such solvates are usually not very useful for pharmaceutical compositions from the point of view of stability of solid form of active substance.
Another example how to increase the solubility of ibandroate in acidic media (content of the stomach) is to use for this dissolution salts with increasing numbers of the neutralised part of bisphosphonic acid.

Literature:
1. Menschutkin N.: Ann Chem Pharm. 1865,133, 317
2. Widler L., et all.: J. Med. Chem. 2002, 45, 3721
3. DE 3623397 (1986)
4. WO 2005/063779
5. WO 2006/002348A2
6. WO 2006/045578A2
7. WO 2006/081962A1
8. WO 2006/081963A1
9. WO 2006/024024A3

### Disclosure of invention

Ibandronate trisodium salt is a good example of an approach how to increase the solubility of ibandronate in acidic media. It is presented in this invention that solid trisodium salt of ibandronate can form several polymorphic forms and also an amorphous form. Ibandronate trisodium salt has less ability to form organic solvents solvates in comparison with the monosodium salt. This ability is very surprising and useful for preparation of stable polymorphs from mixtures of water-miscible organic solvents. There is no competition of water with organic solvents to incorporate into the crystal lattice of ibandronate trisodium. Trisodium ibandronate is also very stable in solid form with mixtures of weak acids. This behaviour is a benefit for preparation of solid dosage forms containing solid organic or inorganic acids.

Accordingly, the present invention provides the trisodium salt of ibandronate characterized by formula (I):

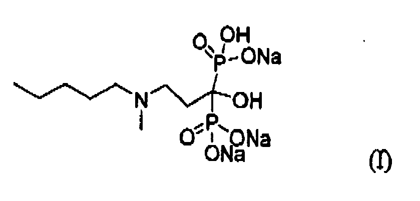

The present invention further provides a process for preparing the trisodium salt of ibandronate of formula (I), which can be depicted by the following scheme:

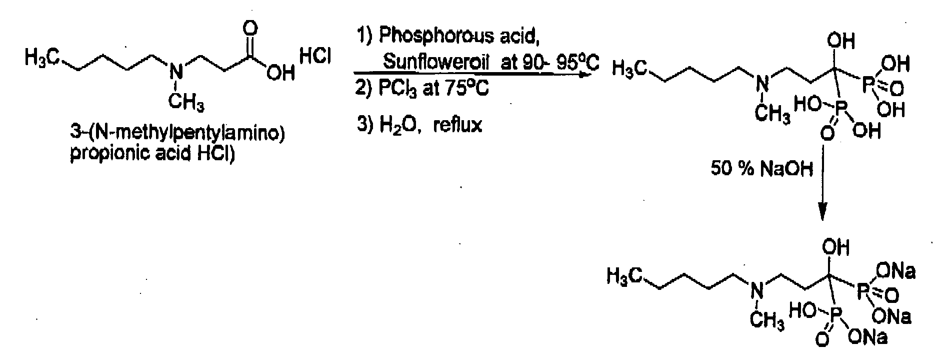

Accordingly, the process of the present invention comprises:
a) mixing 3-(methylpentylamino)propionic acid hydrochloride, phosphorous acid (in a 2.5-3.5 molar excess with respect to 3-(methylpentylamino)propionic acid hydrochloride), sunflower oil (in an amount of 6-9 volume parts for 1 weight part of 3-(methylpentylamino)propionic acid hydrochloride) and phosphorus trichloride (in a 3-4 molar excess with respect to 3-(methylpentylamino)propionic acid hydrochloride);
b) adding water (in an amount of 6-9 volume parts for 1 weight part of 3-(methylpentylamino)propionic acid hydrochloride) and after separation of the phases;
c) adjusting pH of the water phase to pH 12.5;
d) adding a water-miscible co-solvent and cooling down to precipitate the product or evaporate to dryness.

In a specific embodiment, the process of the present invention comprises:
a) mixing 3-(methylpentylamino)propionic acid hydrochloride, phosphorous acid (in a 2.5-3.5 molar excess with respect to 3-(methylpentylamino)propionic acid hydrochloride) and sunflower oil (in an amount of 6-9 volume parts for 1 weight part of 3-(methylpentylamino)propionic acid hydrochloride) at 90-95°C. Upon melting of the mixture, temperature is reduced to 70-75°C and phosphorus trichloride (in a 3-4 molar excess with respect to 3-(methylpentylamino)propionic acid hydrochloride) is added to the reaction mixture. Stirring is carried out at this temperature for 30 minutes and followed by additional stirring for 3-4 hours at 90-95°C;
b) adding water (in an amount of 6-9 volume parts for 1 weight part of 3-(methylpentylamino)propionic acid hydrochloride) and refluxing for another 4 hours;
c) adjusting pH of separated water phase to pH 12.5 with sodium hydroxide; and
d) adding a water-miscible co-solvent and cooling down to precipitate the product or evaporate to dryness..

### The process can be preferably carried out in the following way:

A reaction vessel is charged with 3-(methylpentylamino)propionic acid hydrochloride, phosphorous acid and sunflower oil. The suspension is heated. Temperature is adjusted to 90-95°C at which all components of the reaction medium are provided in liquid form.
A NaOH trap is mounted to the system to catch the possible HCl emission. The reaction mixture is cooled to 70-75°C and at this temperature phosphorus trichloride is added dropwise and stirred for further 30 min. And then the temperature is adjusted to 90-95°C and the reaction medium is stirred for 3-4 hours. Then the reaction medium is allowed to cool down to 10-15°C and water is slowly added to the reaction mixture. The mixture is stirred for 4 hours at reflux temperature. Phases are separated. The water phase is filtered on celite and allowed to cool down to room temperature. The pH is adjusted to 12.5 with 50% NaOH solution.
The solid form was prepared by addition of another organic solvent in which the product is not soluble and isolation of the solid form out or by evaporation of water.

The same process with isolation of ibandronate sodium is possible.
After the phases are separated, the water phase is filtered on celite and allowed to cool down until room temperature. The pH is adjusted to 4.4 with 50% NaOH solution.
Methanol is poured into the aqueous phase containing the crude product at room temperature.
This mixture is stirred for 3 hours for obtaining the thus formed white crystals in precipitated form. The cake of the crystalline precipitate is dissolved in water (at 50-60°C) and then pH is adjusted to 12.5 with 50% NaOH solution. This way trisodium ibandronate in solution was prepared. The solid form was prepared by addition of another solvent and isolation of the solid form or by evaporation of water.

The present invention further provides a process for preparing the trisodium salt of ibandronate, which comprises admixing the monosodium salt of ibandronate or ibandronic acid or their solvates with water or a mixture water/sodium hydroxide and adjustment of pH to 12.5.

The present invention also provides the trisodium salt of ibandronate in the solid form. Preferably, the solid form contains 0.5-30% of water, more preferably, 7-29% of water.

The present invention also provides the trisodium salt of ibandronate in solid crystal form I, characterized by XRPD peaks [°2 θ] (Copper (Cu)) at 4.3, 8.5, 10.5, 12.7, 16.9 ± 0.2 °2θ and by ¹³C ssNMR signals 76.5, 75.5, 74.4, 53.2, 40.7, 30.2, 28.4, 23.6, 22.9, 21.7 and 15.4 ppm.

Said crystal form I can be, according to the present invention, prepared by a process comprising dissolution of monosodium salt of ibandronate or ibandronic acid or their solvates in water with addition of sodium hydroxide, adjustment of pH to 12.5 and addition of ethanol.

The present invention also provides a process for preparing of Ibandronate trisodium in solid crystal form I, comprising dissolution of monosodium salt of ibandronate or ibandronic acid or their solvates in water with addition of sodium hydroxide, adjustment of pH to 12.5 and addition of acetone.

The present invention further provides the trisodium salt of Ibandronate in solid crystal form II characterized by XRPD peaks [°2 θ] (Copper (Cu)) at 3.8, 4.6, 9.4, 10.3, 19.7 ± 0.2 °2θ and by ¹³C ssNMR signals 73.2, 72.9, 71.8, 56.1, 51.3, 37.6, 29.1, 24.8, 22.8, 21.7 and 14.3 ppm.

Said form can be, according to the invention, prepared by a process comprising dissolution of monosodium salt of ibandronate or ibandronic acid or their solvates in water with addition of sodium hydroxide, adjustment of pH to 12.5, addition of ethanol and stirring at room temperature.

The present invention further provides the trisodium salt of Ibandronate in semicrystal form I characterized by XRPD peaks [°2 θ] (Copper (Cu)) at 3.9 and 4.4.± 0.2 °2 θ.

Said form can be, according to the invention, prepared by a process comprising dissolution of monosodium salt of ibandronate or ibandronic acid or their solvates in water solution of sodium hydroxide, adjustment of pH to 12.5 and evaporation of water.

The present invention further provides a process for preparing of Ibandronate trisodium in semicrystal form I, comprising the exposition of Ibandronate trisodium salt crystal form I to temperature 130°C.

The present invention further provides the trisodium salt of Ibandronate in semicrystal form II characterized by XRPD peaks [°2 θ] (Copper(Cu)) at 4.0, 8.0, 12.8 and a broad peak at 16.2 ± 0.2 °2θ.

Said form can be, according to the invention, prepared by a process comprising dissolution of monosodium salt of ibandronate or ibandronic acid or their solvates in water and addition of sodium hydroxide, adjustment of pH to 12.5 and addition of 2-propanol.

The present invention further provides the trisodium salt of Ibandronate in semicrystal form III characterized by XRPD peaks [°2 θ] (Copper (Cu)) at 4.15, 5.4, 10.1, 12.7, 19.4, 20.9 and 33.4.± 0.2 °2θ.

Said form can be, according to the invention, prepared by a process comprising dissolution of monosodium salt of ibandronate or ibandronic acid or their solvates in water and adjustment of pH to 12.5 with sodium hydroxide and addition of ethanol or 2-propanol and a cooling step to temperature below 0°C.

The present invention further provides the trisodium salt of Ibandronate in semicrystal form IV characterized by XRPD peaks [°2 θ] (Copper (Cu)) at 3.4, 6.7, 13.7, 16.8, 20.9 ± 0.2 °2θ.

Said form can be, according to the invention, prepared by a process comprising dissolution of monosodium salt of ibandronate or ibandronic acid or their solvates in water and adjustment of pH to 12.5 with sodium hydroxide and addition of acetone and stirring at the room temperature.

The present invention further provides the trisodium salt of Ibandronate in amorphous form characterized by XRPD with a very broad peak between 15 to 25 °2θ (Copper(Cu)).

Said form can be, according to the invention, prepared by a process comprising exposing of ibandronate trisodium semicrystaline form in air with RH 20-90%.

The invention further provides a process for preparing of Ibandronate trisodium in amorphous form, comprising exposing of ibandronate trisodium semicrystaline form I in air with RH 30-60%.

The present invention further provides a pharmaceutical composition of ibandronate trisodium salts comprising a solid organic acid and pharmaceutically acceptable excipients. Preferably, the solid organic acid is fumaric acid.

### Brief description of drawings

Fig. 1 depicts the CP-MAS ¹³C NMR and XRPD spectra of crystal form I of trisodium ibandronate prepared in Example 2.
Fig. 2 depicts the CP-MAS ¹³C NMR and XRPD spectra of crystal form II of trisodium ibandronate prepared in Example 3.
Fig. 3 depicts the CP-MAS ¹³C NMR and XRPD spectra of crystal form I of trisodium ibandronate prepared in Example 4.
Fig. 4 depicts the CP-MAS ¹³C NMR 4 spectrum of semicrystalline form I of trisodium ibandronate prepared in Example 5.
Fig. 5 depicts the XRPD spectra of semicrystalline form I of trisodium ibandronate prepared in Examples 5 and 6.
Fig. 6 depicts the CP-MAS ¹³C NMR and XRPD spectra of semicrystalline form II of trisodium ibandronate prepared in Example 7.
Fig. 7 depicts the XRPD spectra of the amorphous form of trisodium ibandronate prepared in Example 8.
Fig. 8 depicts the XRPD spectrum of semicrystalline form III of trisodium ibandronate prepared in Example 9.
Fig. 9 depicts the XRPD spectrum of semicrystalline form IV of trisodium ibandronate prepared in Example 10.
Fig. 10 depicts the XRPD spectrum of semicrystalline form III of trisodium ibandronate prepared in Example 11.
Fig. 11 depicts the XRPD spectrum of semicrystalline form III of trisodium ibandronate prepared in Example 12.
Fig. 12 depicts the ¹³C NMR spectra of ibandronate trisodium in admixture with fumaric acid in comparison with those of fumaric acid, fumaric acid monosodium salt and fumaric acid disodium salt.

The following examples explain the process in more detail.

### Example 1:

A reaction vessel is charged with 3-(methylpentylamino)propionic acid hydrochloride (21 g, 100 mmol), phosphorous acid (23 g, 280 mmol) and sunflower oil (170 ml). The suspension is heated. Temperature is adjusted to 90-95°C at which all components of the reaction medium are provided in liquid form.

A NaOH trap was mounted to the system to catch the possible HCl emission. The reaction mixture was cooled to 70-75°C and at this temperature phosphorus trichloride (29 ml, 330 mmol) was added dropwise and stirred for further 30 min. And then the temperature was adjusted to 90-95°C and the reaction medium was stirred for 3-4 hours. Then the reaction medium was allowed to cool down to 10-15°C and water (170 ml) slowly added to the reaction mixture. The mixture was stirred for 4 hours at reflux temperature. Phases are separated. The water phase was filtered on celite and allowed to cool down to room temperature. The pH was adjusted to 4.4 using 50% NaOH solution. Methanol (200 ml) was poured into the aqueous phase containing the crude product. This mixture was stirred for 3 hours for obtaining the thus formed white crystals in precipitated form. Ibandronic acid monosodium salt was filtered and dried yielding 25 g of Ibandronic acid monosodium salt.

### Example 2:

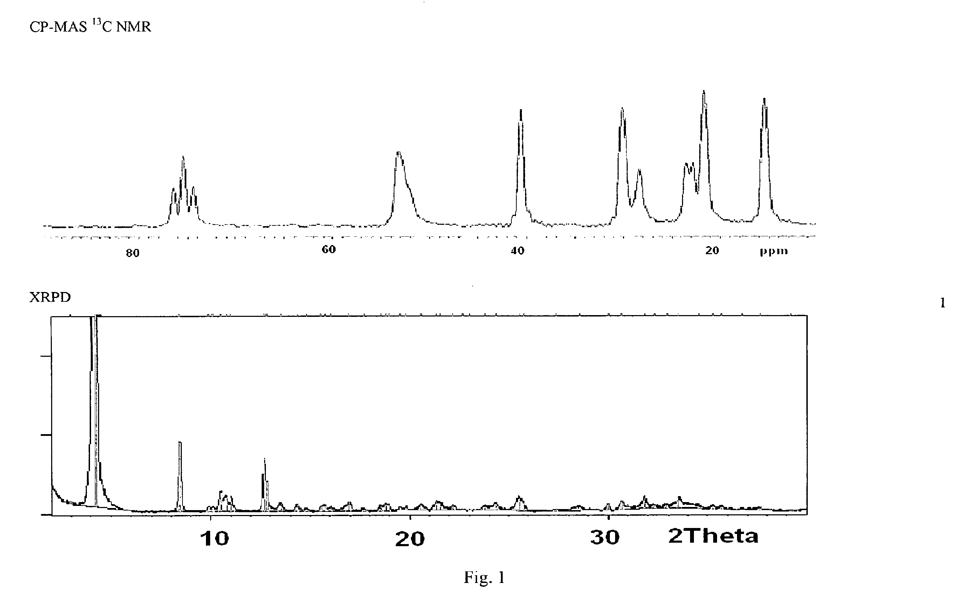

The ibandronic acid monosodium salt (10 g) (obtained according to reference example 1) and NaOH (2.3 g) was dissolved in water (50 ml) and the pH was adjusted to 12.5. The solution was filtered and ethanol (50 ml) was poured into this solution at room temperature. This mixture was cooled to -12 °C for 20 hours. Then the mixture was stayed at room temperature for 4 days for obtaining thus formed white crystals. The product was filtered and dried. It was obtained 7.8 g of trisodium ibandronate in crystal form I (29% water w/w).
(sample 43-188-3)
CP-MAS ¹³C NMR and XRPD spectra are in Fig. 1.

### Example 3:

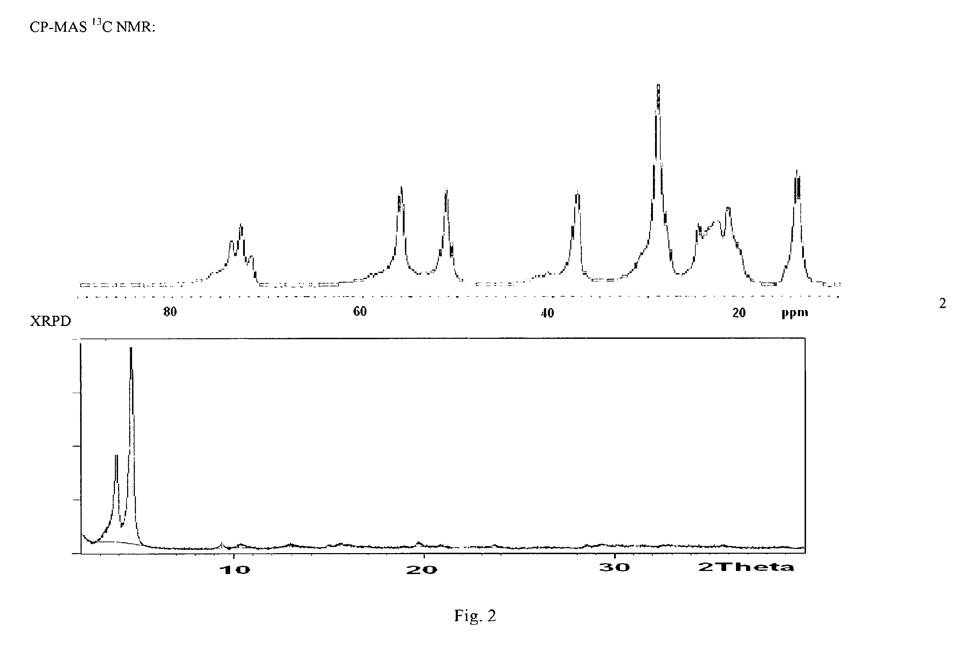

The ibandronic acid monosodium salt (10 g) (obtained according to reference example 1) and NaOH (2.3 g) was dissolved in water (50 ml) and the pH was adjusted to 12.5. The solution was filtered and ethanol (75 ml) was poured into this solution at room temperature. This mixture was stirred for 3 hours for obtaining the thus formed white crystals. Product was filtered and dried. It was obtained 11 g of trisodium ibandronate in crystal form II. (18% water w/w). (sample 43-176-6)
CP-MAS ¹³C NMR and XRPD spectra are in Fig. 2.

### Example 4:

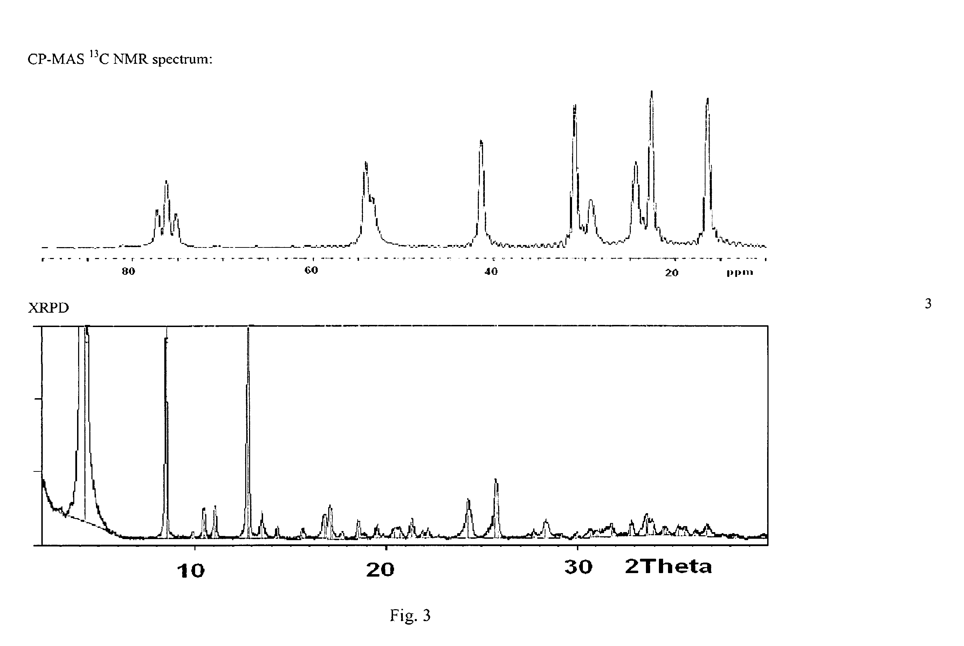

The ibandronic acid monosodium salt (10 g) (obtained according to reference example 1) and NaOH (2.3 g) was dissolved in water (50 ml) and the pH was adjusted to 12.5. The solution is filtered and acetone (100 ml) was poured into this solution at room temperature. This mixture was stirred for 3 hours for obtaining the thus formed white crystals. The product was filtered and dried to obtain 10.2 g of trisodium ibandronate in crystal form I (26% water w/w). (sample 43-185-5)
CP-MAS ¹³C NMR and XRPD spectra are in Fig. 3.

### Example 5:

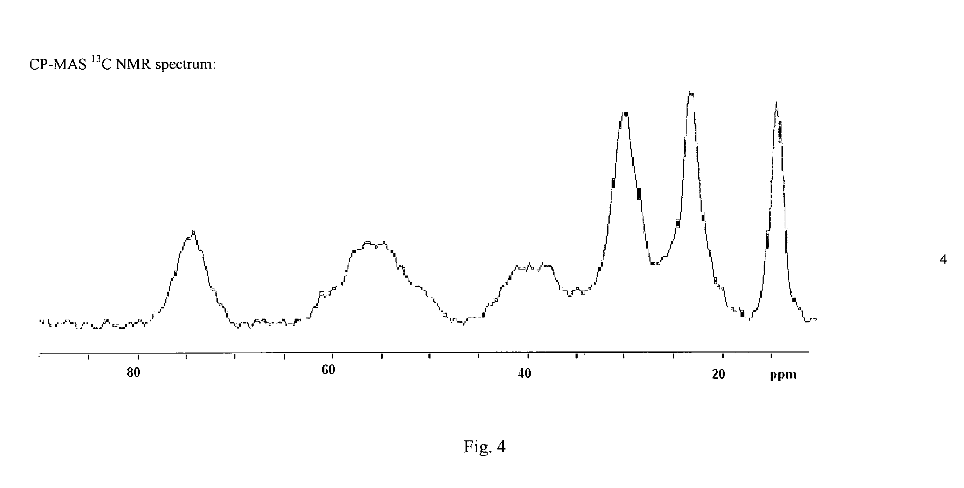

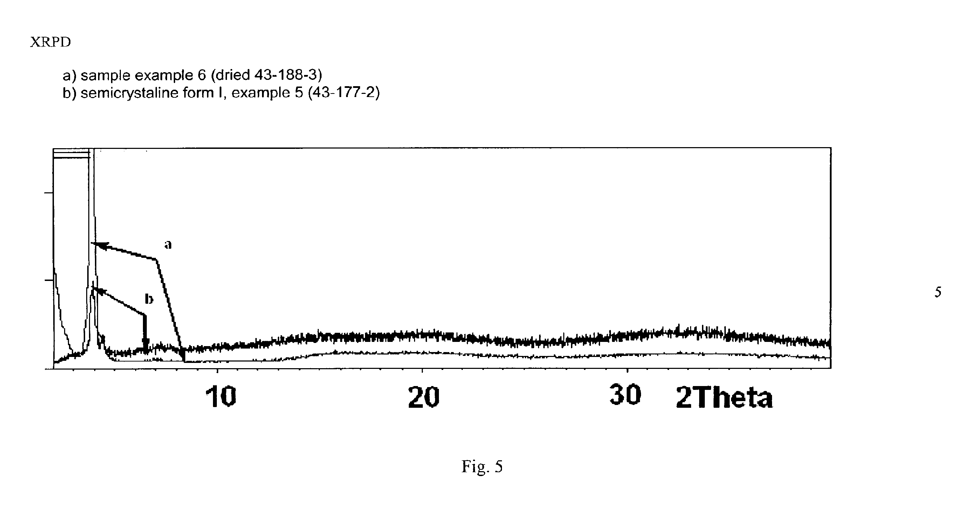

The ibandronic acid monosodium salt (10 g) (obtained according to reference example 1) was dissolved in 0.46 M NaOH (125 ml) and the pH was adjusted to 12.5. The solution was filtered and the solution was evaporated to dryness to obtain 11 g of trisodium ibandronate in semicrystalline form I (16% water w/w) (sample 43-177-2)
CP-MAS ¹³C NMR spectrum is in Fig. 4.
The XRPD spectrum is in Fig. 5.

### Example 6:

The ibandronic acid trisodium salt form I (1 g) (obtained according to reference example 2) was dried at 130°C for Ih. Form I was transformed to semicrystalline form I (0.78 g).
The XRPD spectrum is in Fig. 5.

### Example 7:

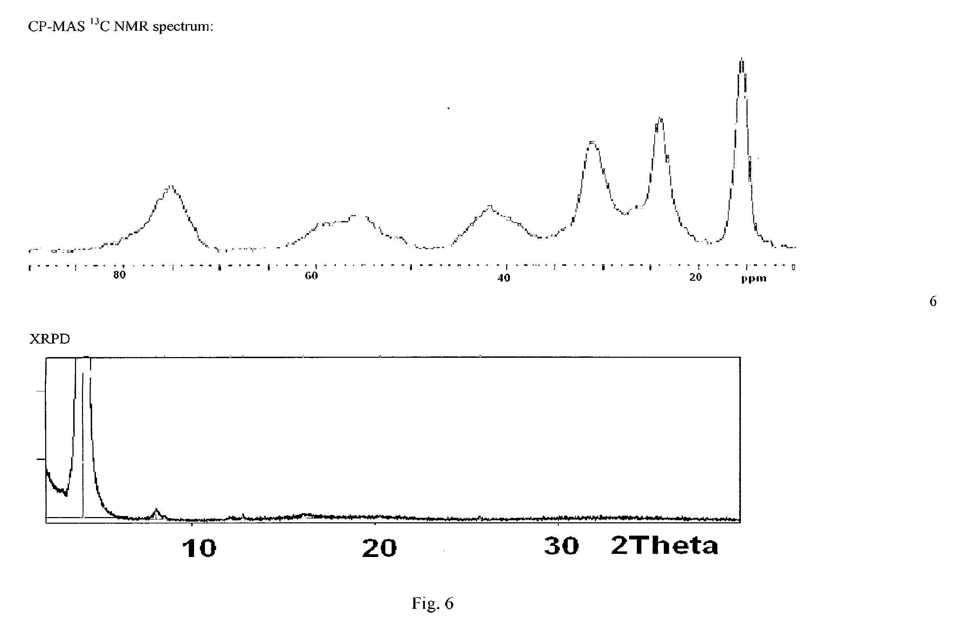

The ibandronic acid monosodium salt (10 g) (obtained according to reference example 1) and NaOH (2.3 g) was dissolved in water (50 ml) and the pH was adjusted to 12.5. The solution was filtered and 2-propanol (125 ml) was poured into this solution at room temperature. This mixture was stirred for 3 hours for obtaining the so formed white precipitate. The product was filtered and dried to obtain 10 g of trisodium ibandronate in semicrystalline form II (8% water w/w). (sample 43-185-3)
CP-MAS ¹³C NMR and XRPD spectra are in Fig. 6.

### Example 8

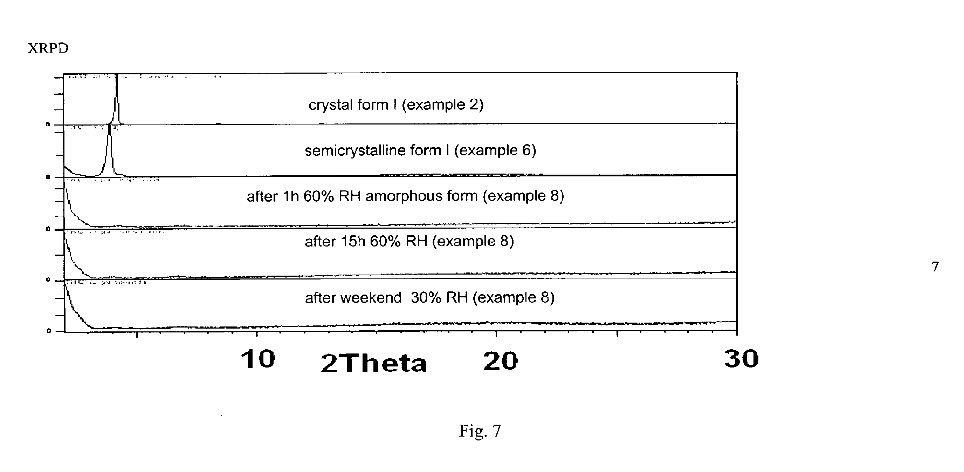

Trisodium ibandronate semicrystaline form I (0.5g) (obtained according to reference example 6) was allowed to stand at 60% R.H at 25°C for 15 hours to form amorphous form of trisodium ibandronate and later to stand at the 30% RH and temperature 25°C for two days resulting in amorphous form of ibandronate trisodium (0.61g) (29% water w/w)
XRPD spectra are in Fig. 7.

### Example 9

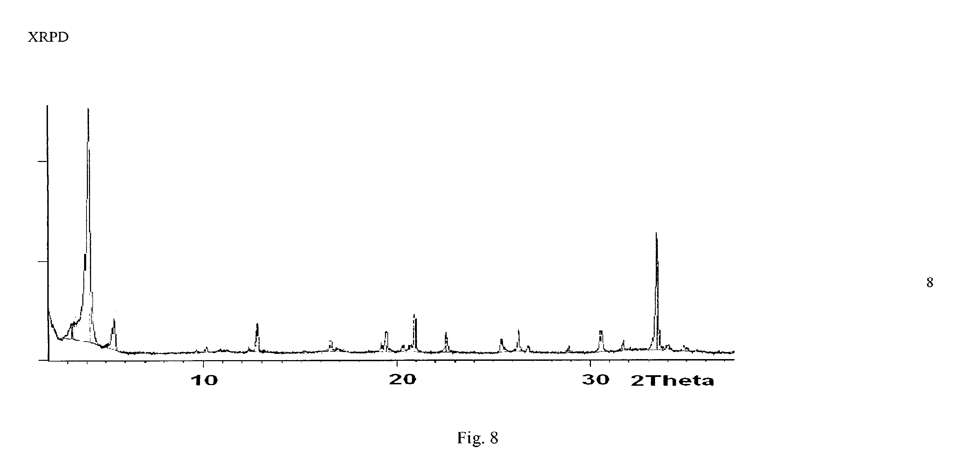

Reaction vessel is charged with 3-(methylpentylamino)propionic acid HCl (7.1 g) and phosphorous acid (8 g) and sunflower oil (50 ml). Temperature is adjusted to 90-95°C at which all components of the reaction medium are provided in liquid form. A NaOH trap is mounted to the system to catch the possible HCl emission. The reaction mixture is cooled to 70-75°C and at this temperature phosphorus trichloride (10 ml) is added dropwise and stirred for further 30 min. And then the temperature is adjusted to 90-95°C and the reaction medium is stirred for 3-4 hours. Then the reaction medium is allowed to cool down until 10-15°C and water (50 ml) is slowly added to the reaction mixture. The mixture is stirred for 4 hours at reflux temperature. Phases are separated. The water phase is filtered on celite and allowed to cool down to room temperature. The pH is adjusted to 12.5 with 50% NaOH solution (10 ml) and NaOH (9 g). The filtration of the solution was followed with the addition of 100 ml of ethanol at room temperature. This mixture was kept at -12°C for 20 hours. This mixture is stirred for 48 hours at room temperature for obtaining the thus formed white solid in precipitated form. Ibandronate triodium salt was filtered and dried (70°C, 6 h) yielding 8 g of Ibandronate trisodium. This sample was semicrystalline form III and contained 12% of water (TGA)
CP-MAS ¹³C NMR spectrum does not differ from semicrystalline form I or II.
XRPD spectrum is in Fig. 8.

### Example 10

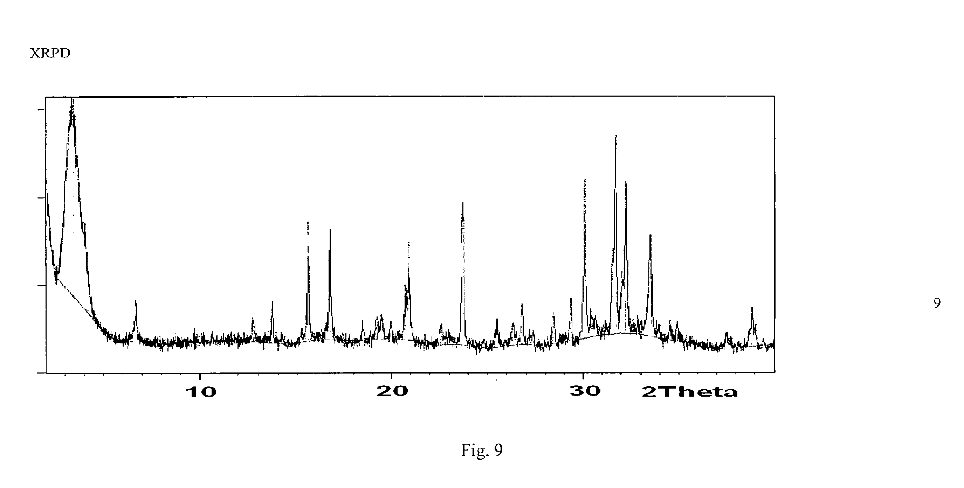

Reaction vessel is charged with 3-(methylpentylamino)propionic acid HCl (14.2 g) and phosphorous acid (16 g) and sunflower oil (100 ml). Temperature is adjusted to 90-95°C at which all components of the reaction medium are provided in liquid form. A NaOH trap is mounted to the system to catch the possible HCl emission. The reaction mixture is cooled to 70-75°C and at this temperature phosphorus trichloride (20 ml) is added dropwise and stirred for further 30 min. And then the temperature is adjusted to 90-95°C and the reaction medium is stirred for 3-4 hours. Then the reaction medium is allowed to cool down until 10-1 5°C and water (100 ml) is slowly added to the reaction mixture. The mixture is stirred for 4 hours at reflux temperature. Phases are separated. The water phase is filtered on celite and allowed to cool down to room temperature. The pH is adjusted to 12.5 with 50% NaOH solution (20 ml) and NaOH (17 g). The solution was filtered and acetone (300 ml) was poured into this solution at room temperature. The mixture was also stirred for 16 h at room temperature. White solid is formed after seeding with the trace of ibandronate trisodium prepared in example 9. Ibandronate triodium salt was filtered and dried (70°C, 6 h) yielding 9.2 g of Ibandronate trisodium. This sample was semicrystalline form IV and contained 22% of water (TGA). CP-MAS ¹³C NMR spectrum does not differ from semicrystalline form I or II.
XRPD spectrum is in Fig. 9.

### Example 11

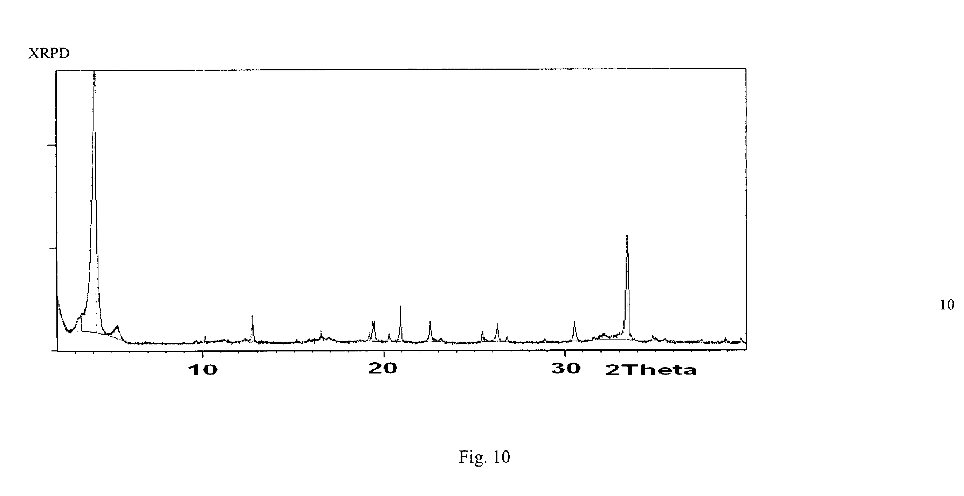

Reaction vessel is charged with 3-(methylpentylamino)propionic acid HCl (14.2 g) and phosphorous acid (16 g) and sunflower oil (100 ml). Temperature is adjusted to 90-95°C at which all components of the reaction medium are provided in liquid form. A NaOH trap is mounted to the system to catch the possible HCl emission. The reaction mixture is cooled to 70-75°C and at this temperature phosphorus trichloride (20 ml) is added dropwise and stirred for further 30 min. And then the temperature is adjusted to 90-95°C and the reaction medium is stirred for 3-4 hours. Then the reaction medium is allowed to cool down to 10-15°C and water (100 ml) is slowly added to the reaction mixture. The mixture is stirred for 4 hours at reflux temperature. Phases are separated. The water phase is filtered on celite and allowed to cool down until room temperature. The pH is adjusted to 12.5 with 50% NaOH solution (20 ml) and NaOH (17 g). The solution was filtered and ethanol (500 ml) was poured into this solution at room temperature. This mixture is stirred for 14 hours for obtaining the thus formed white solid in precipitated form. Ibandronate triodium salt was filtered and dried (70°C, 6 h) yielding 9.4 g of Ibandronate trisodium. This sample was semicrystalline form III and contained 11.3% of water (TGA)
CP-MAS ¹³C NMR spectrum does not differ from semicrystalline form I or II.
XRPD spectrum is in Fig. 10.

### Example 12

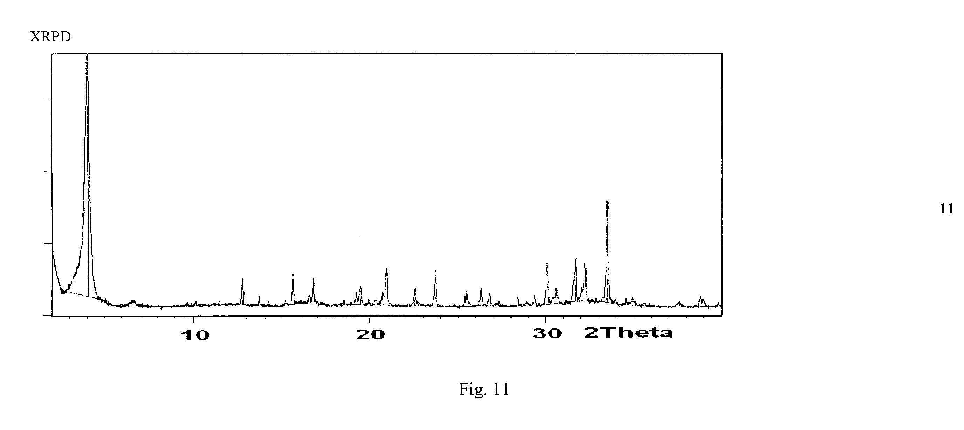

Reaction vessel is charged with 3-(methylpentylamino)propionic acid HCl (14.2 g) and phosphorous acid (16 g) and sunflower oil (100 ml). Temperature is adjusted to 90-95°C at which all components of the reaction medium are provided in liquid form. A NaOH trap is mounted to the system to catch the possible HCl emission. The reaction mixture is cooled to 70-75°C and at this temperature phosphorus trichloride (20 ml) is added dropwise and stirred for further 30 min. And then the temperature is adjusted to 90-95°C and the reaction medium is stirred for 3-4 hours. Then the reaction medium is allowed to cool down to 10-15°C and water (100 ml) is slowly added to the reaction mixture. The mixture is stirred for 4 hours at reflux temperature. Phases are separated. The water phase is filtered on celite and allowed to cool down to room temperature. The pH is adjusted to 12.5 with 50% NaOH solution (20 ml) and NaOH (17 g). The solution was filtered and isopropyl alcohol IPA (300 ml) was poured into this solution at room temperature. The mixture was also stirred for 16 h at room temperature. White solid was precipitated after the seeding with trace of the solid prepared in example 9. Ibandronate triodium salt was filtered and dried (70°C, 6 h) yielding 9.1 g of Ibandronate trisodium. This sample was semicrystalline form III and contained 15.1% of water (TGA)
CP-MAS ¹³C NMR spectrum does not change from semicrystalline form I or II.
XRPD spectrum is in Fig. 11.

### Example 13

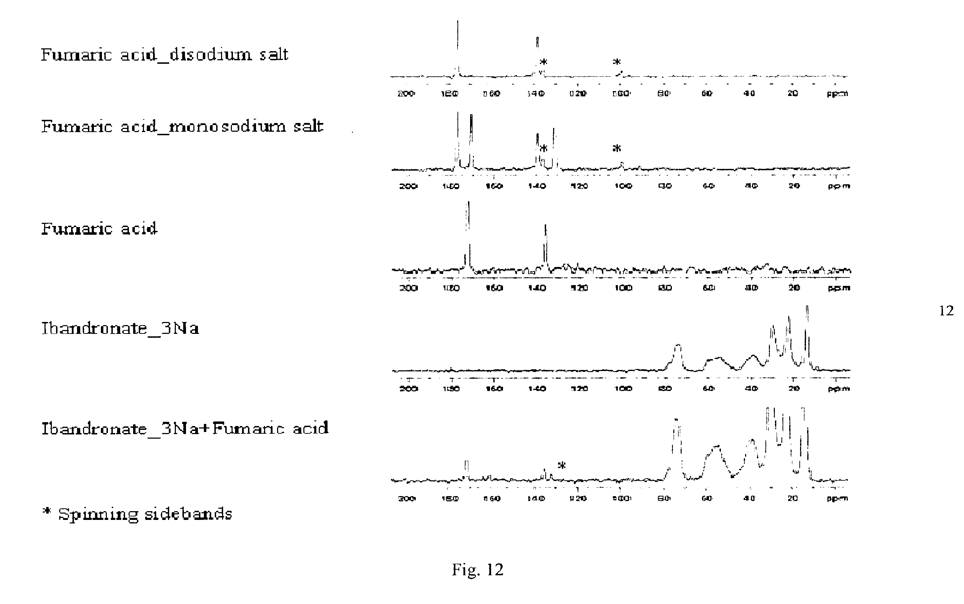

Ibandronate trisodium in semicrystalline form I (containing 12% of water) 439 mg and fumaric acid 116 mg was treated in solid form in a mortar for 5 minutes and then filled to the 7 mm rotor for measuring the solid state NMR and measuring the ¹³C NMR spectrum. Comparing the spectra of both components and fumaric acid monosodium salt and fumaric acid disodium salt it is obvious that both components in the solid state are remaining in their original form and no neutralization or changing the solid state form occurred. See Figure 12.

## Claims

1. The trisodium salt of Ibandronate **characterized by** formula (I)

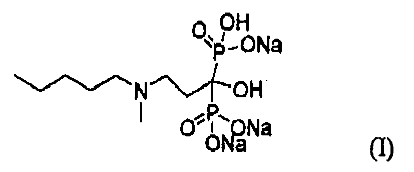

2. A process for preparing the trisodium salt of Ibandronate defined in claim 1, comprising
a) mixing 3-(methylpentylamino)propionic acid hydrochloride, phosphorous acid (in a 2.5-3.5 molar excess with respect to 3-(methylpentylamino)propionic acid hydrochloride), sunflower oil (in an amount of 6-9 volume parts for 1 weight part of 3-(methylpentylamino)propionic acid hydrochloride) and phosphorus trichloride (in a 3-4 molar excess with respect to 3-(methylpentylamino)propionic acid hydrochloride);
b) adding water (in an amount of 6-9 volume parts for 1 weight part of 3-(methylpentylamino)propionic acid hydrochloride) and after separation of the phases;
c) adjusting pH of the water phase to pH 12.5;
d) adding a water-miscible co-solvent and cooling down to precipitate the product or evaporate to dryness.

3. The process as defined in claim 2, comprising
a) mixing 3-(methylpentylamino)propionic acid hydrochloride, phosphorous acid (in a 2.5-3.5 molar excess with respect to 3-(methylpentylamino)propionic acid hydrochloride) and sunflower oil (in an amount of 6-9 volume parts for 1 weight part of 3-(methylpentylamino)propionic acid hydrochloride) at 90-95°C; upon melting of the mixture, reducing the temperature to 70-75°C, adding phosphorus trichloride (in a 3-4 molar excess with respect to 3-(methylpentylamino)propionic acid hydrochloride) is added to the reaction mixture and stirring at this temperature for 30 minutes and followed by additional stirring for 3-4 hours at 90-95°C;
b) adding water (in an amount of 6-9 volume parts for 1 weight part of 3-(methylpentylamino)propionic acid hydrochloride) and refluxing for another 4 hours;
c) adjusting pH of separated water phase to pH 12.5 with sodium hydroxide;
d) adding a water-miscible co-solvent and cooling down to precipitate the product or evaporate to dryness.

4. A process for preparing the trisodium salt of Ibandronate defined in claim 1, comprising admixing the monosodium salt of ibandronate or ibandronic acid or their solvates with water or a mixture water/sodium hydroxide and adjustment of pH to 12.5.

5. The trisodium salt of Ibandronate of claim 1 in the solid form.

6. The trisodium salt of Ibandronate in the solid form containing 0.5-30% of water.

7. The trisodium salt of Ibandronate in the solid form containing 7-29% of water.

8. The trisodium salt of Ibandronate in solid crystal form L, **characterized by** XRPD peaks [°2θ] (Copper (Cu)) at 4.3, 8.5, 10.5, 12.7, 16.9 ± 0.2 °2θ and by ¹³C ssNMR signals 76.5, 75.5, 74.4, 53.2, 40.7, 30.2, 28.4, 23.6, 22.9, 21.7 and 15.4 ppm.

9. A process for preparing of Ibandronate trisodium in solid crystal form I **characterized in** claim 8, comprising dissolution of monosodium salt of ibandronate or ibandronic acid or their solvates in water with addition of sodium hydroxide, adjustment of pH to 12.5 and addition of ethanol.

10. A process for preparing of Ibandronate trisodium in solid crystal form I **characterized in** claim 8, comprising dissolution of monosodium salt of ibandronate or ibandronic acid or their solvates in water with addition of sodium hydroxide, adjustment of pH to 12.5 and addition of acetone.

11. The trisodium salt of Ibandronate in solid crystal form II **characterized by** XRPD peaks [°2θ] (Copper (Cu)) at 3.8, 4.6, 9.4, 10.3, 19.7 ± 0.2 °2θ and by ¹³C ssNMR signals 73.2, 72.9, 71.8, 56.1, 51.3, 37.6, 29.1, 24.8, 22.8, 21.7 and 14.3 ppm.

12. A process for preparing of Ibandronate trisodium in solid crystal form II **characterized in** claim 11, comprising dissolution of monosodium salt of ibandronate or ibandronic acid or their solvates in water with addition of sodium hydroxide, adjustment of pH to 12.5, addition of ethanol and stirring at room temperature.

13. The trisodium salt of Ibandronate in semicrystal form I **characterized by** XRPD peaks [°2 θ] (Copper (Cu)) at 3.9 and 4.4.± 0.2 °2 θ.

14. A process for preparing of Ibandronate trisodium in somicrystal form I **characterized in** claim 13, comprising dissolution of monosodium salt of ibandronate or ibandronic acid or their solvates in water solution of sodium hydroxide, adjustment of pH to 12.5 and evaporation of water.

15. A process for preparing of Ibandronate trisodium in semicrystal form I, comprising the exposition of ibandronate trisodium salt crystal form I to temperature 130°C.

16. The trisodium salt of Ibandronate in semicrystal form II **characterized by** XRPD peaks [°2θ] (Copper(Cu)) at 4.0, 8.0, 12.8 and a broad peak at 16.2 ± 0.2 °2θ.

17. A process for preparing of Ibandronate trisodium in semicrystal form II **characterized in** claim 16, comprising dissolution of monosodium salt of ibandronate or ibandronic acid or their solvates in water and addition of sodium hydroxide, adjustment of pH to 12.5 and addition of 2-propanol.

18. The trisodium salt of Ibandronate in semicrystal form III **characterized by** XRPD peaks [°2θ] (Copper (Cu)) at 4.15, 5.4, 10.1, 12.7, 19.4, 20.9 and 33.4.± 0.2 °2θ.

19. A process for preparing of Ibandronate trisodium in semicrystal form III **characterized in** claim 18, comprising dissolution of monosodium salt of ibandronate or ibandronic acid or their solvates in water and adjustment of pH to 12.5 with sodium hydroxide and addition of ethanol or 2-propanol and a cooling step to temperature below 0°C.

20. The trisodium salt of Ibandronate in semicrystal form IV **characterized by** XRPD peaks [°2θ] (Copper (Cu)) at 3.4, 6.7, 13.7, 16.8, 20.9 ± 0.2 °2θ.

21. A process for preparing of Ibandronate trisodium in semicrystal form IV **characterized in** claim 20, comprising dissolution of monosodium salt of ibandronate or ibandronic acid or their solvates in water and adjustment of pH to 12.5 with sodium hydroxide and addition of acetone and stirring at the room temperature.

22. The trisodium salt of Ibandronate in amorphous form **characterized by** XRPD with a very broad peak between 15 to 25 °2θ (Copper(Cu)).

23. A process for preparing of Ibandronate trisodium in amorphous form, comprising exposing of ibandronate trisodium semicrystaline form in air with RH 20-90%.

24. A process for preparing of Ibandronate trisodium in amorphous form, comprising exposing of ibandronate trisodium semicrystaline form I in air with RH 30-60%.

25. A pharmaceutical composition of ibandronate trisodium salts comprising a solid organic acid and pharmaceutically acceptable excipients.

26. The composition of claim 25, wherein the solid organic acid is fumaric acid.

## Amended claims

### Amended claims in accordance with Rule 137(2) EPC.

**1.** A process for preparing the ibandronic acid **characterized by** formula (I) 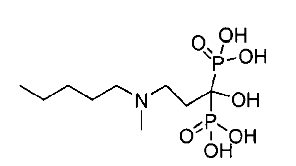 in the form of the monosodium or trisodium salt, comprising the following steps :
a) mixing 3-(methylpentylamino)propionic acid hydrochloride, phosphorous acid (in a 2.5-3.5 molar excess with respect to 3-(methylpentylamino)propionic acid hydrochloride), sunflower oil (in an amount of 6-9 volume parts for 1 weight part of3-(methylpentylamino)propionic acid hydrochloride) and phosphorus trichloride (in a 3-4 molar excess with respect to 3-(methylpentylamino)propionic acid hydrochloride);
b) adding water (in an amount of 6-9 volume parts for 1 weight part of 3-(methylpentylamino)propionic acid hydrochloride) and after separation of the phases;
c) adjusting pH of the water phase to pH =4.4 in the case of monosodium salt or adjusting pH of the water phase to pH =12.5 in the case of trisodium salt;
d) adding a water-miscible co-solvent and cooling down to precipitate the product or evaporate to dryness.

**2.** A process for preparing the trisodium salt of Ibandronate, comprising admixing the monosodium salt of ibandronate or ibandronic acid or their solvates with water or a mixture water/sodium hydroxide and adjustment of pH to 12.5.

**3.** The process according to claims 1 or 2, comprising the following steps:
a) mixing 3-(methylpentylamino)propionic acid hydrochloride, phosphorous acid (in a 2.5-3.5 molar excess with respect to 3-(methylpentylamino)propionic acid hydrochloride) and sunflower oil (in an amount of 6-9 volume parts for 1 weight part of 3-(methylpentylamino)propionic acid hydrochloride) at 90-95°C; upon melting of the mixture, reducing the temperature to 70-75°C, adding phosphorus trichloride (in a 3-4 molar excess with respect to 3-(methylpentylamino)propionic acid hydrochloride) is added to the reaction mixture and stirring at this temperature for 30 minutes and followed by additional stirring for 3-4 hours at 90-95°C;
b) adding water (in an amount of 6-9 volume parts for 1 weight part of 3-(methylpentylamino)propionic acid hydrochloride) and refluxing for another 4 hours;
c) adjusting pH of the water phase to pH =4.4 in a case of monosodium salt or to pH =12.5 in case of trisodium salt with sodium hydroxide;
d) adding a water-miscible co-solvent and cooling down to precipitate the product or evaporate to dryness.

**4.** A pharmaceutical composition of ibandronic acid or its sodium salt prepared by the process **characterized in** any one of claims 1-3.

**5.** A pharmaceutical composition of ibandronic acid or its sodium salt prepared by the process **characterized in** any one of claims 1-3, further comprising a solid organic acid and pharmaceutically acceptable excipients.

**6.** The composition of claim 5, wherein the solid organic acid is fumaric acid.
